# EUROPÄISCHE PATENTSCHRIFT

(11) **EP 0 595 297 B1**
(45) Veröffentlichungstag und Bekanntmachung des Hinweises auf die Patenterteilung: **18.07.2001**
(21) Anmeldenummer: 93117414.8
(22) Anmeldetag: 27.10.1993
(51) Int. Cl.: A61K 35/78

(54) **Ligninpolymerzusammensetzung zur Behandlung von Hautproblemen**
Lignin polymer composition for the treatment of skin problems
Composition de polymères de lignine pour le traitement des problèmes de la peau

(30) Priorität: 28.10.1992 DE 4236346
(43) Veröffentlichungstag der Anmeldung: 04.05.1994
(73) Patentinhaber: Mach, Chantal, D-85614 Kirchseeon (DE)
(72) Erfinder: Mach, Walter, Dr., D-85614 Kirchseeon (DE); Mach, Chantal, Dr., D-85614 Kirchseeon (DE)
(74) Vertreter: Freiherr von Wittgenstein, Arved, Dr.

(56) Entgegenhaltungen:
- DE-A- 4 017 091

## Beschreibung

Die Erfindung betrifft eine Wirkstoffgruppe zur äußerlichen Behandlung der Haut und ihrer Krankheiten, ihre Herstellung sowie ihre Verwendung.

Dabei wird hier unter Haut auch die Schleimhaut verstanden. Die Versorgung des Organs Haut mit dessen Alterungserscheinungen mildernden bzw. bekämpfenden Stoffen sowie die Bekämpfung von Hautkrankheiten, wie Neurodermatitis und Psoriasis sowie Infektionen der Haut durch Bakterien und Viren, wie Herpes, Akne, oxidative Schäden und UV-Schäden gestaltet sich schwierig.

Es wurde bereits vorgeschlagen, Vitamin A und Vitamin E in Hautcremes zur Verbesserung des Hautbildes und zur Anregung der Zellfunktion bzw. Schutz der Zellfunktion vor den sog. freien Radikalen einzusetzen. Insbesondere Vitamin A wird schon seit längerer Zeit bei der Bekämpfung der Akne oder aber auch allgemein zur Verbesserung des Hautbildes eingesetzt.

Ein weiteres Problem ist die Infektion der Haut mit Viren, die Warzen (e.g. Papillomaviren), Bläschen (Herpes) hervorrufen.

Weiterhin wird das Erscheinungsbild der Haut auch häufig durch allergische oder entzündliche Reaktionen ungünstig verändert, wie Psoriasis oder Neurodermatitis.

Die bisher bekannten Mittel zur Verbesserung des Hautbildes und zur Bekämpfung von Infektionen der Haut, auch der Schleimhäute, waren insofern nicht zufriedenstellend, als sie entweder Nebenwirkungen aufwiesen und insbesondere bei einem Gehalt an Eiweißen zu Allergien Anlaß geben konnten, oder aber es traten Gewöhnungs- oder Nebeneffekte auf, die eine Langzeitanwendung dieser Materialien erschwerten.

Es ist Aufgabe der Erfindung, einen Wirkstoff zu finden, der eine Neutralisation bzw. Bekämpfung von Haut-Infektionen ohne zytotoxische Effekte und ohne unerwünschte immunologische Reaktionen im Organismus des behandelten Patienten auszulösen, bewirkt.

Die Aufgabe wird erfindungsgemäß gelöst durch eine Wirkstoffgruppe nach Patentanspruch 1.

Dadurch, daß die erfindungsgemäße Wirkstoffgruppe proteinfrei ist, ist eine Ausbildung von Allergien gegen Eiweißstoffe vermieden. Die erfindungsgemäße Wirkstoffgruppe ist ferner dazu befähigt, freie Radikale zu binden, die als Zellgifte ebenfalls das Erscheinungsbild der Haut und deren Widerstandsfähigkeit negativ beeinflussen. In überraschender Weise konnten antiphlogistische, heilungsfördernde, antiallergische Eigenschaften auch bei Psoriasis und Neurodermatitis beobachtet werden.

Vorteilhafte Weiterbildungen des Patentanspruches 1 ergeben sich aus den Unteransprüchen.

Die zu extrahierenden holzartigen Materialien können ausgewählt werden aus der Gruppe bestehend aus:
natives Holz, wie bspw. Kernholz von Bäumen in Form von "milled wood", Weichholz, Hartholz, holzartige Substanzen, wie sie bspw. in Kernobst- oder Nußschalen auftreten, allgemein verholzte Pflanzenbestandteile, bevorzugt auch als entharzte Ausgangsprodukte; Gräser, wie Esparto, etc.; biotechnologisch darstellbare holzige Substanzen durch Aufbereitung pflanzlicher Zellkulturen;
synthetische Holz-Analoga über Darstellung von FREUDENBERG-Dehydrierungspolymerisaten von Mono- und/oder Dilignolen u. dgl. und Aufpfropfen der Dehydrierungspolymerisate auf Polysaccharide; Lignin-Polysaccharid-Komplexe, (erhältlich durch alkalische Extraktion von Chlorit-Holzzellulose);
sowie Mischungen derselben.
Vorteilhafterweise werden als Basen KOH, NaOH, LiOH und Ammoniak verwendet, es können aber auch andere Basen eingesetzt werden.

Die wäßrige Lösung der Wirkstoffgruppe wird in an sich bekannter Weise, ggf. mit Hilfsstoffen, zu einer fließfähigen pharmazeutischen oder kosmetischen Zubereitung verarbeitet, je nach Anwendungsform, bspw. mit einer Salbengrundlage zu einer Salbe zur äußeren Anwendung oder mit einer Basislösung zu einer Lösung zur äußeren Anwendung oder auch in an sich bekannter Form zu Wirkstoffampullen, die vor Gebrauch geöffnet werden.

Für kosmetische Zwecke können auch an sich bekannte Stabilisatoren und Konservierungsmittel, bevorzugt Lebensmittelkonservierungs-mittel, eingesetzt werden.

Vorteilhafte Weiterbildungen der Erfindung ergeben sich aus den Unteransprüchen.

Besondere Vorteile der erfindungsgemäßen Wirkstoffgruppe sind:
- Auch bei Langzeitbehandlung (s.c.) über Jahre tritt keine unerwünschte Sensibilisierung auf, es fehlt jede Allgemeinreaktion auf die Medikation wie reaktives Fieber u.ä., das Blutbild wird nicht medikationsabhängig in irgend einer Weise verschlechtert.
- Bei Infektionskrankheiten der Haut durch konventionelle Viren (Herpes, Papillomaviren u.s.w.) kommt es zu einer raschen und vollkommenen Restitution ohne unerwünschte Nebenwirkungen
- da es sich um einen non-protein-Wirkstoff handelt, wirkt er nicht als Reizstoff.
- selbst bei extremster überdosierung (z.B. 48 mg/24 Std, wiederholt) kommt es weder zu akuten noch zu chronischen Folgeschäden.

Primäre Schwerpunkt-Indikationsgebiete für das neue Wirkstoffsystem sind - ohne allerdings eine Einschränkung auf diese Gebiete zu beabsichtigen:
- Virus- und Bakterien - Infektionen und Läsionen der Haut
- Zellschädigungen der Haut allgemein

Das erfindungsgemäße Extraktionsprodukt entwickelt gemäß den durchgeführten Untersuchungen einerseits eine entzündungshemmende Wirkung und ist andererseits praktisch nebenwirkungsfrei.

Ein Vorteil der erfindungsgemäßen, Zuckerketten aufweisenden lignoide Materialien aufweisenden Wirkstoffgruppe ist, daß es sich um ein "non-protein" handelt, das die Immunabwehr gegen körperfremde Eiweißstoffe nicht irritiert und daher für eine Langzeitanwendung auf der Haut ohne Allergiebildungen geeignet ist.

Es wird angenommen, daß die erfindungsgemäße Wirkstoffgruppe die Vermehrung von Viren an der Hautoberfläche, insbesondere hier eines Herpes-Virus, verhindert.

Durch wissenschaftliche Untersuchungen konnte festgestellt werden, daß die Wirkung der Wirkstoffgruppe auf beschädigte oder angegriffene Epidermis-Zellen überraschend signifikant ist und keine wesentlichen Nebenwirkungen toxischer Art eintreten.

Demzufolge ist die erfindungsgemäße Wirkstoffgruppe für eine Langzeitmedikation geeignet; es tritt bei Langzeitgabe kein Wirkungsverlust auf; es kann auch im Verdachtsfall ohne Risiko eingesetzt werden und ist mit geeigneten Wirkstoffen unspezifischer Art kombinierbar.

Nachfolgend soll anhand von Ausführungsbeispielen sowie der begleitenden Zeichnung die Erfindung näher erläutert werden. Dabei zeigt:

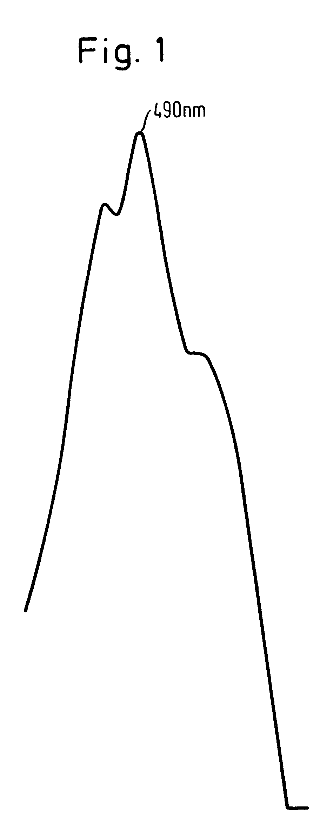

- Fig. 1:: Fluoreszenzspektren von Lösungen der erfindungsgemäßen Wirkstoffgruppe in Wasser

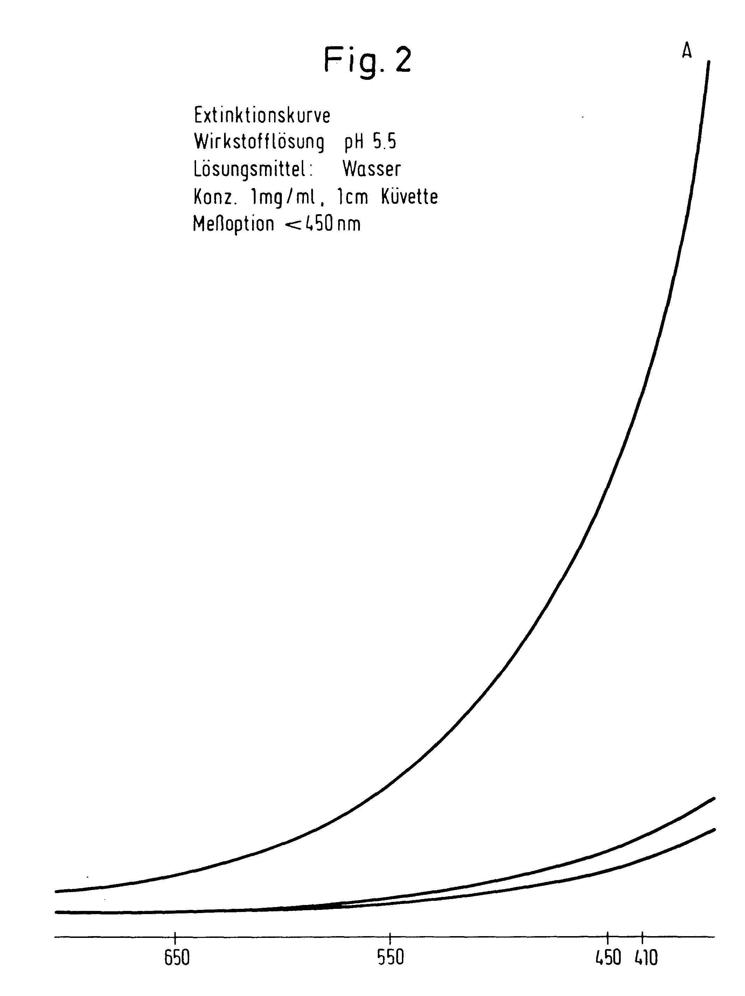

- Fig. 2: UV-Spektrum der Wirkstofflösung

### Beispiel 1

### Herstellung einer erfindungsgemäßen Wirkstoffgruppe

300 g gemahlenes Kernholz der sibirische Lärche (Larix sibiriaca) werden als milled wood in 6000 ml entmineralisiertem Wasser durch Rühren dispergiert und 150 g KOH zugegeben. Dieser Ansatz wird bei Raumtemperatur 30 Tage stehengelassen. Anschließend werden die Feststoffe über eine Nutsche abgetrennt, der Rückstand verworfen und das Filtrat blank zentrifugiert. Anschließend wird durch Ultrafiltration an alkalifesten Ultrafiltrationsmembranen mit einem nominellen Trennschnitt von 70 000 Dalton eine blanke gelbe Lösung gewonnen und das Retentat verworfen. Die blanke gelbe Lösung wurde mit einem Kationenaustauscherharz in der Wasserstoff-Form auf pH-Wert 5 angesäuert.

Von dieser blanken gelben Matrix-Lösung wird nun eine Probe von 3 ml entnommen, in eine Quarzküvette (1cm) gegeben und ein Fluoreszenzanregungsspektrum mit einer Anregungswellenlänge von 257 nm registriert. Es zeigte sich ein typisches Maximum mit drei Schultern mit einer Spitze bei 490 nm Emissionswellenlänge.

### Beispiel 2

### Herstellung einer erfindungsgemäßen Wirkstoffgruppe

210 g Kernholz der Lärche, 60 g Kernholz der Buche und 30 g Fruchtschalenholz von prunus avium wurden, wie in Beispiel 1 angegeben, verarbeitet.

### Beispiel 3

### Herstellung einer erfindungsgemäßen Wirkstoffgruppe

In 25 Liter entmineralisiertes Wasser werden 500 g pulverförmiges Cortex Cinnamonum ceylanicum und 15,5 g NaOH dispergiert bzw. gelöst. Die Weiterverarbeitung erfolgte wie in Beispiel 1.

Allgemein ist festzustellen, daß die Holzextraktionsbedingungen einen großen Einfluß auf die molekulare Struktur der löslichen Polysaccharid-Lignineinheiten haben. Die molekulare Struktur der Matrix hängt dabei in vielfältiger Weise von Ausgangsmaterial ab, also davon, welches Kernholz eines Baumes, ob Stroh oder Gräser Verwendung findet.

Dabei ist auch die Holzart (Nadelbaum, Laubbaum) und auch das als Ausgangsmaterial eingesetzte Pflanzenteil (Nußschalen, Pfirsichkerne, Weichholzteile) bestimmend; so ist bspw. die Bindung zwichen P und L bei Nußschalen erheblich fester als bei einem Weichholz).
Ferner ist für das Produkt die Methode der Extraktion wesentlich, dabei sind insbesondere geeignet: alkalische Extraktion; Behandlung mit heißem Wasser/Wasserdampf unter Druck (Autoklaven) oder auch Elektrolyse mit NaCl als Elektrolyt.
Die erfindungsgemäße Wirkstoffgruppe enthält sehr viele Struktureinheiten aus dem Bereich der Naturstoffe Arene und Kohlenhydrate.

Nachfolgend sollen nun mit Material des Beispiels 3 an verschiedenen Zellsystemen durchgeführte Untersuchungen aufgeführt werden.

### Testreihe

### Test auf antiphlogistische Wirksamkeit einer Wirkstoff enthaltenden Salbe am Histamin-Rattenpfotenödem

SPF-Wistarratten wurden zur ödembildung mit 0.2 ml einer 0.1%-igen Histaminlösung in physiol. NaCl s.c. behandelt.
Unmittelbar nach der ödemprovokation wurden die Tiere immobilisiert und die behandelten Pfoten der Kontrolltiere ständig in Wasser und die der Testgruppe in eine 0,4%-ige Lösung (berechnet als Trockensubstanz) der Wirkstofflösung des Beispiels 3 getaucht. Die Ablesungen der Pfotenvolumina erfolgten sofort nach Provokation und im 1-Stundenabstand bis zu 3 Std. nach der Provokation.
Die Volumenmessungen zeigten bei der mit Wirkstoff behandelten Rattengruppe eine deutliche linear zeitabhängige Volumenverminderung, d.h. nach 3 Std. war das Pfotenödem hochsignifikant zurückgegangen.

### Therapeutische Prüfung am gereizten Kaninchenauge

Es wurden 6 gesunde Albino-Kaninchen verwendet. Vor und nach der Behandlung wurden die Augen aller Tiere mit 2%-igem Fluoreszein unter UV-Licht untersucht, um jede nicht sichtbare Läsion auszuschließen.

Es wurde 0,1 ml einer 1%igen Histaminlösung in den Konjunktivalsack des linken Kaninchenauges appliziert. Das rechte Auge blieb unbehandelt und diente als Kontrolle. Bei drei der 6 behandelten Tiere wurden gleichzeitig 0.1 ml einer 1%-igen Wirkstofflösung mit einem pH-Wert von 7, die sterilisiert war, appliziert.

Unter den gegebenen Testbedingungen wurde festgestellt, daß durch Applikation von Histamin eine Augenveränderung hervorgerufen wird. Wird gleichzeitig die Wirkstofflösung appliziert, tritt keine oder eine nur geringgradige und kurzfristige Schwellung der Konjunktiva auf. Damit wurde für die Wirkstofflösung ein schleimhautabschwellender Effekt festgestellt.

### Antioxidative Wirkung

Es wurde versucht, die Toxizität von Sauerstoffspezies, die beim Bestrahlen einer stark, verdünnten Wasserstoffperoxidlösung (0,01 ppm H₂O₂ mit UV-Strahlung entstehen, auf Daphnien durch Zugabe der Wirkstofflösung nach Beispiel 3 zu verhindern.

Dabei zirkulierte eine Nährlösung in einer Bestrahlungsapparatur, die im Kühler auf 18 bis 20°C konstant gehalten wurde. Gleichzeitig erfolgte eine gute Sättigung mit Luftsauerstoff. Der Nährlösung wurden 0,12 ppm Wasserstoffperoxid zugegeben. Dies ist eine für Daphnia magna unschädliche Dosis. Durch Einschalten einer groß dimensionierten Bestrahlungsanlage konnte in der Nährlösung die Konzentration an toxischen Sauerstoffspezies angehoben werden. Zur Kontrolle wurde während des Versuchs die Redoxspannung verfolgt. Die durch die Bestrahlung in das System eingebrachte Wärme wurde durch die Kühlung abgeführt, so daß eine Erhöhung der Toxizität durch Temperatureinfluß vermieden wurde. Bei der Zugabe von Wirkstofflösung zum System konnte die Beweglichkeit der Daphnien signifikant gegenüber Systemen ohne die Wirkstofflösung erhalten werden.

## Patentansprüche

1. Wirkstoffgruppe insbesondere zur Behandlung der Haut und zur Unterstützung der Heilung von Hautproblemen, erhältlich durch
Extraktion von Holz oder holzartigen Materialien, eingeschlossen pflanzliche Zellkulturen holzartiger Materialien in wäßrigem Medium in alkalischem Milieu bei einem pH-Wert von etwa 8 bis 14 etwa 30 Tage bei Raumtemperatur:
Abtrennen der unlöslichen festen Bestandteile; Ultrafiltration des erhaltenen Materials bei nominellen Trennschnittwerten von unter 100kD, bevorzugt unter 70 kD, wobei das Retentat verworfen wird,
und ggf. Einstellen der ultrafiltrierten Wirkstofflösung auf einen hautaffinen pH-Wert

2. Wirkstoffgruppe nach Anspruch 1, dadurch gekennzeichnet, daß die zu extrahierenden holzartigen Materialien ausgewählt sind aus der Gruppe bestehend aus:
nativem Holz, biotechnologisch darstellbaren holzigen Substanzen durch Aufbereitung pflanzlicher Zellkulturen, synthetischen Holz-Analoga nach Darstellung von Freudenberg-Dehydrierungspolymerisaten (DHP) von Mono- und oder Dilignolen und Aufpfropfen der DHP's auf Polysaccharide; sowie Lignin-Polysaccharid-Komplexen, erhältlich durch alkalische Extraktion von Chlorit-Holzzellulose; sowie Mischungen derselben.

3. Wirkstoffgruppe nach einem der vorangehenden Ansprüche, dadurch gekennzeichnet, daß die in den einzelnen Verfahrensschritten eingesetzten Basen KOH, NAHO, LiOH und Ammoniak sind.

4. Wirkstoffgruppe nach einem der vorangehenden Ansprüche, dadurch gekennzeichnet, daß die wäßrige Lösung des Produkts in an sich bekannter Weise, ggf. mit Hilfsstoffen, zu einer fließfähigen Zubereitung verarbeitet wird.

5. Verfahren zur Herstellung einer Wirkstoffgruppe nach einem der vorangehenden Ansprüche insbesondere zur Behandlung der Haut und zur Unterstützung der Heilung von Hautproblemen, gekennzeichnet durch die Schritte:
Extraktion von Holz oder holzartigen Materialien oder pflanzlicher Zellkulturen holzartigen Materialien in wäßrigem Medium in alkalischem Milieu bei einem pH-Wert von etwa 8 bis 14 etwa 30 Tage bei Raumtemperatur:
Abtrennen der unlöslichen festen Bestandteile;
Ultrafiltration des erhaltenen Materials bei nominellen Trennschnittwerten von unter 100 kD, bevorzugt unter 70 kD, wobei das Retentat verworfen wird, und ggf. Einstellen der ultrafiltrierten Wirkstofflösung auf einen hautaffinen pH-Wert ggf. Weiterverarbeitung zu auf die Haut auftragbaren fließfähigen Zubereitungen in an sich bekannter Weise.

6. Verwendung der Wirkstoffgruppe nach einem der vorangehenden Ansprüche zur Herstellung eines Mittels zur Anwendung in der Kosmetik und der Human- oder Veterinärmedizin.

7. Verwendung nach Anspruch 6 zur Bekämpfung von Irritationen und Infektionen der menschlichen und/oder tierischen Haut, bspw. virale und bakterielle Infektionen, wie Herpes, Akne, Neurodermatitis, Psoriasis, Verbrennungen, eingeschlossen Sonnenbrand.

## Claims

1. Group of active substances, more especially for treating the skin and for promoting the healing of skin problems, obtainable by:
extracting wood or wood-like materials, including vegetable cell cultures of wood-like materials in an aqueous medium in an alkaline environment at a pH value of substantially between 8 and 14 for substantially 30 days at ambient temperature;
separating the insoluble, solid ingredients; ultrafiltering the obtained material at nominal cross-sectional values of less than 100 kD, preferably less than 70 kD, the residue being rejected;
and possibly adjusting the ultrafiltered solution of active substances to a pH value having affinity with the skin.

2. Group of active substances according to claim 1, characterised in that the wood-like materials to be extracted are selected from the group including: native wood, biotechnologically preparable wooden substances by processing vegetable cell cultures, synthetic wood analogs in accordance with the preparation of Freudenberg dehydrogenating polymerides (DHP) from mono- and/or dilignols and grafting the DHPs on polysaccharides; as well as lignin polysaccharide complexes, obtainable by alkaline extraction of chlorite wood cellulose; as well as mixtures thereof.

3. Group of active substances according to one of the preceding claims, characterised in that the bases used in the individual method steps are KOH, NAHO, LiOH and ammonia.

4. Group of active substances according to one of the preceding claims, characterised in that the aqueous solution of the product is processed in a manner known per se, possibly with adjuvants, to form a flowable preparation.

5. Method of producing a group of active substances according to one of the preceding claims, more especially for treating the skin and for promoting the healing of skin problems, characterised by the steps:
extracting wood or wood-like materials or vegetable cell cultures of wood-like materials in an aqueous medium in an alkaline environment at a pH value of substantially between 8 and 14 for substantially 30 days at ambient temperature;
separating the insoluble, solid ingredients;
ultrafiltering the obtained material at nominal cross-sectional values of less than 100 kD, preferably less than 70 kD, the residue being rejected; and
possibly adjusting the ultrafiltered solution of active substances to a pH value having affinity with the skin; and
possibly further processing such to form flowable preparations, which can be applied to the skin, in a manner known per se.

6. Use of the group of active substances according to one of the preceding claims for producing an agent for application in cosmetics and in human or veterinary medicine.

7. Use according to claim 6. for combatting irritations and infections of human and/or animal skin, e.g. viral and bacterial infections, such as herpes, acne, neurodermatitis, psoriasis, bums, including sunburn.

## Revendications

1. Groupe de matières actives, en particulier pour le traitement de la peau et pour faciliter le soin de problèmes de peau, obtenu par extraction de bois ou de matières du type bois, y compris des cultures cellulaires de plantes de matières du type bois dans un agent aqueux dans un milieu alcalin à une valeur de pH d'environ 8 à 14 environ 30 jours à température ambiante ; séparation des constituants solides insolubles ; ultrafiltration de la matière obtenue pour des valeurs de seuil nominales inférieures à 100 kD, avantageusement inférieures à 70 kD, le rétentat étant rejeté ; et, le cas échéant, réglage de la solution ultrafiltrée de matières actives à une valeur de pH affine à la peau.

2. Groupe de matières actives selon la revendication 1,
caractérisé en ce que les matières du type bois à extraire sont choisies à partir du groupe constitué : du bois natif, des substances ligneuses pouvant être réalisées de façon biotechnologique par préparation de cultures cellulaires de plantes, des analogues synthétiques du bois suivant la préparation de polymères de déshydrogénation de Freudenberg (DHP) de mono- et/ou dilignoles, et implantation des DHP sur des polysaccharides ; ainsi que des complexes de lignine-polysaccharide, obtenus par extraction alcaline de cellulose de bois au chlorite ; ainsi que des mélanges de ceux-ci.

3. Groupe de matières actives selon une des revendications précédentes,
caractérisé en ce que les bases utilisées dans les étapes individuelles du procédé sont KOH, NAHO, LiOH et l'ammoniaque.

4. Groupe de matières actives selon une des revendications précédentes,
caractérisé en ce que la solution aqueuse du produit est traitée, de façon connue en soi, le cas échéant avec des matières consommables, en une préparation fluide.

5. Procédé pour fabriquer un groupe de matières actives selon une des revendications précédentes, en particulier pour le traitement de la peau et pour faciliter le soin de problèmes de peau,
caractérisé par les étapes :
extraction de bois ou de matières du type bois ou de cultures cellulaires de plantes de matières du type bois dans un agent aqueux dans un milieu alcalin à une valeur de pH d'environ 8 à 14 environ 30 jours à température ambiante ;
séparation des constituants solides insolubles ;
ultrafiltration de la matière obtenue pour des valeurs de seuil nominales inférieures à 100 kD, avantageusement inférieures à 70 kD, le rétentat étant rejeté, et
le cas échéant, réglage de la solution ultrafiltrée de matières actives à une valeur de pH affine à la peau,
le cas échéant, traitement ultérieur en des préparations fluides pouvant être appliquées sur la peau, d'une façon connue en soi.

6. Utilisation du groupe de matières actives selon une des revendications précédentes pour fabriquer un agent utilisable en cosmétique et en médecine humaine ou vétérinaire.

7. Utilisation selon la revendication 6 pour combattre des irritations et infections de la peau humaine et/ou animale, par exemple des infections virales et bactériennes, comme l'herpès, l'acné, la neurodermatose, le psoriasis, des brûlures, y compris des coups de soleil.
